# EUROPÄISCHE PATENTANMELDUNG

(11) **EP 0 697 390 A1**
(43) Veröffentlichungstag der Anmeldung: **21.02.1996**
(21) Anmeldenummer: 95112168.0
(22) Anmeldetag: 02.08.1995
(51) Int. Cl.: C07C 45/45, C07C 49/784, C07C 49/76, C08K 5/07, C07C 45/57, C07C 45/59, C07C 45/60

(54) **Verfahren zur Herstellung von 1,3-Diketonen und deren Verwendung als Stabilisatoren für Kunststoffe**

(30) Priorität: 03.08.1994 DE 4427512
(71) Anmelder: BASF AKTIENGESELLSCHAFT, D-67056 Ludwigshafen (DE)
(72) Erfinder: Meixner, Hubert, Dr., D-67069 Ludwigshafen (DE); Reuther, Wolfgang, Dr., D-69118 Heidelberg (DE); Hahn, Erwin, Dr., D-69118 Heidelberg (DE); Königstein, Volker, Dr., D-67133 Maxdorf (DE)
(74) Vertreter: Geissler, Bernhard, Dr.

(57) **Zusammenfassung**

Lineare 1,3-Diketone, bei denen die Substituenten C₁-C₂₀-Alkyl, Phenyl und dergleichen sind, werden durch Claisen-Kondensation von entsprechenden Ketonen mit entsprechenden Estern in Gegenwart eines Alkali- oder Erdalkalimetallhydrids oder C₁-C₅-Alkali- oder Erdalkalimetallalkoholats als Base und in einem inerten Lösungsmittel oder Lösungsmittelgemisch bei einer Temperatur im Bereich von 95 - 150 °C hergestellt.

## Beschreibung

Die vorliegende Erfindung bezieht sich auf ein Verfahren zur Herstellung von 1,3-Diketonen nach der Claisen-Kondensation. Weiterhin bezieht sich die Erfindung auf die Verwendung des organischen Reaktionsproduktes dieser Reaktion für die Stabilisierung von Kunststoffen. Gegenstand der Erfindung ist auch eine Stabilisatorzusammensetzung für Kunststoffe, insbesondere für halogenhaltige Kunststoffe wie Polyvinylchlorid (PVC).

1,3-Diketone wie beispielsweise Dibenzoylmethan sind als Zusätze für PVC bekannt. In der EP-A-454 624 sind Verfahren zur Herstellung solcher 1,3-Diketone sowie deren Verwendung in PVC beschrieben. Die Umsetzung nach der Claisen-Kondensation erfolgt dabei bei niedrigen Temperaturen von 0 - 30 °C (Beispiel 1), 15 °C bzw. 30 °C (Beispiel 2), 0 - 20 °C (Beispiel 3), 15 - 30 °C (Beispiel 4 der EP-A-454 624).

Festes Dibenzoylmethan, wie es z. B. als "Rhodiastab® 83" im Handel erhältlich ist, wird durch zeitlich aufwendige Reinigung einschließlich Waschen, Kristallisieren und Rekristallisieren hergestellt.

Die Herstellung von 1,3-Diketonen für die kommerzielle Anwendung als Kunststoffstabilisatoren nach dem Stande der Technik ist aufwendig und kostspielig.

Eine Aufgabe der vorliegenden Erfindung besteht daher darin, 1,3-Diketon-Stabilisatoren zur Verfügung zu stellen, die in der Herstellung einfacher und kostengünstiger sind. Darüber hinaus sollen vorzugsweise 1,3-Diketon-Stabilisatorprodukte geschaffen werden, die eine möglichst hohe, vorzugsweise eine nahezu unbegrenzte Lagerstabilität aufweisen und insbesondere nicht lichtempfindlich sind, kein Nachdunkeln der Produkte aufweisen und somit eine Aufbewahrung in lichtgeschützten Räumen bzw. Behältern vermeidlich werden lassen. Letzteres ist bei der Anwendung der Materialien in PVC-Folien von besonderer Bedeutung, da sonst die Anfangsfarbe sowie die Farbhaltung der PVC-Folien leiden kann, wenn derart gealtertes Material verwendet wird.

Erfindungsgemäß wird ein Verfahren zur Herstellung von 1,3-Diketonen zur Verfügung gestellt, das von einem Claisen-Kondensationsverfahren ausgeht, wie es ausführlich in der EP-A-454 624 beschrieben ist. Das erfindungsgemäße Verfahren zeichnet sich dadurch aus, daß die Keton-/ Esterkondensation bei Temperaturen im Bereich von 95 - 150 °C erfolgt.

Das Verfahren gemäß der Erfindung ist im Patentanspruch 1 definiert. Auf die dortigen Formeln, Definitionen und Abkurzungen wird im folgenden Bezug genommen.

Erfindungsgemäß ist es bevorzugt, eine oder mehrere der folgenden Verfahrensstufen oder Verfahrensmaßnahmen durchzufuhren bzw. in einem oder mehreren der folgenden Verfahrensparameterbereiche zu arbeiten:
- Das gebildete R₅OH wird aus dem Reaktionsgemisch entfernt, vorzugsweise abdestilliert.
- Das gebildete Salz wird in die freie Säure der Formel (I) überführt. Vorzugsweise geschieht dies durch Behandlung des Reaktionsgemisches mit einer starken Säure, insbesondere mit Schwefelsäure.
- Die dadurch entstandenen Nebenprodukte werden vorzugsweise von den organischen Reaktionsprodukten getrennt.
- Das entstandene organische Reaktionsprodukt wird ohne Hochreinigung bzw. Isolierung als ungereinigtes Produkt gewonnen.
- Das im wesentlichen ungereinigte organische Reaktionsprodukt wird zu einem Flüssigstabilisatorbestandteil dadurch weiterverarbeitet, daß diesem ungereinigten organischen Reaktionsprodukt ein chelatbildendes Metallsalz und gegebenenfalls ein Lösungsmittel, insbesondere Toluol oder Xylol, zugesetzt werden.
- Das chelatbildende Metallsalz wird in einer Menge verwendet, so daß das Mol-Verhältnis chelatbildendes Metallsalz zu 1,3-Diketon im Bereich 0:1 - 1:3,3, vorzugsweise 1:2 liegt.

Erfindungsgemäß wird auch ein neuer Co-Stabilisator für Flüssigstabilisatorenanwendung zur Verfügung gestellt. Dieser 1,3-Diketon-Co-Stabilisator zeichnet sich dadurch aus, daß er im wesentlichen aus dem organischen Teil einer Claisen-Kondensation, wie sie im Oberbegriff des Anspruchs 1 definiert ist, ohne Hochreinigung - oder Endreinigung entstehen. Es wurde gefunden, daß diese ungereinigten Materialien für den angegebenen Zweck gut brauchbar sind und wegen der erheblich preiswerteren Herstellung eine sehr attraktive Alternative zu den hochgereinigten bisher eingesetzten und im Handel erhältlichen 1,3-Diketonen darstellen.

Schließlich wird erfindungsgemäß auch ein Kunststoffmaterial zur Verfügung gestellt, das einen Co-Stabilisator, wie er hierin definiert ist, enthält. Die bevorzugte Zusammensetzung des Co-Stabilisators ist wie oben angegeben. Der bevorzugte Kunststoff, der in diesen Kunststoffmaterialien zum Einsatz kommt, ist ein halogenhaltiger Kunststoff, insbesondere ein chlorhaltiger Kunststoff, besonders PVC. Der hierin definierte Co-Stabilisator kommt in dem Kunststoffmaterial gemäß der Erfindung, vorzugsweise in einer Menge von 1 - 7 Gewichtsteilen, bezogen auf 100 Gewichtsteile Kunststoff, zum Einsatz.

Weiterhin stellt die Erfindung ein Verfahren zur Verfügung, bei dem die Alkali-, Erdalkalimetallchelate sowie die Chelate der Metalle der 3. Haupt- und 2. Nebengruppe der 1,3-Diketone, insbesondere die Barium-, Calcium- und Zinkchelate der 1,3-Diketone, in situ gebildet werden. Bei diesem Verfahren wird das hierin definierte ungereinigte organische Reaktionsprodukt mit den oben genannten chelatbildenden Metallsalzen vermischt und zusammen mit den weiteren Stabilisatorbestandteilen dem Kunststoff beigegeben. Die bevorzugten Kunststoffe sind die oben definierten halogenhaltigen, insbesondere die chlorhaltigen Kunststoffe, ganz besonders PVC. Es wird durch diese erfindungsgemäße Verarbeitungsweise in doppelter Weise ein signifikanter technischer Fortschritt erzielt: Zum einen werden nämlich die zahlreichen, komplizierten und teuren Reinigungsschritte für das 1,3-Diketon eingespart, zum anderen muß das 1,3-Diketon nicht erst noch bei dessen Einsatz gelöst und zu Metallchelatkomplexen verarbeitet werden. Vielmehr geschieht all dies vorzugsweise in einem einzigen Schritt bzw. einer Verfahrensstufe.

Weitere bevorzugte Ausführungsformen der Erfindung ergeben sich aus den Ansprüchen sowie den folgenden Beispielen.

Hauptbestandteile eines typischen, erfindungsgemäßen Stabilisators sind
1 - 5 Gewichtsteile 1,3-Diketon bzw. entsprechender Menge Metalldiketonat
5 - 50 Gewichtsteile Bariumseife einer C₈- bis C₁₈-Carbonsäure
2 - 35 Gewichtsteile Zinkseife einer C₈- bis C₁₈-Fettsäure
0 - 55 Gewichtsteile organisches Phosphit
Weitere Zuschläge wie Epoxide, Weichmacher, Antioxidantien und Lösemittel können eingesetzt werden.

Zur Darstellung eines typischen, erfindungsgemäßen Flüssigstabilisators werden 3% des 1,3-Diketonats Dibenzoylmethan als 18%-ige Lösung in Xylol sowie 40% C₈- bis C₁₈-Fettsäuren vorgelegt und bei erhöhter Temperatur (90 bis 130 °C) mit den Metallhydroxiden und -oxiden (Barium- und Zinkverbindungen) zu den Metallseifen und Chelatkomplexen umgesetzt, wobei Reaktionswasser und Lösemittel abdestilliert werden. Nach Zugabe des Phosphits und Filtration der Lösung über Kieselgel verbleibt der Flüssigstabilisator als klare niederviskose Flüssigkeit.

Zur Herstellung einer losung von 1,3-Diketon in Xylol geht man folgendermaßen vor:
Unter Stickstoffatmosphäre werden in wasserfreiem Xylol der Methylester einer Carbonsäure und die zu verwendende Base gelöst und unter Erhitzen portionsweise mit der stöchiometrischen Menge an Acetophenon zum 1,3-Diekton-Salz umgesetzt. Das freie 1,3-Diketon läßt sich daraus durch Behandeln mit verdünnter Mineralsäure und anschließendem Abtrennen der wäßrigen Phase als gesättigte 18%-ige Xylollösung erhalten.

Die folgende Tabelle zeigt verwendbare Ester, Lösemittel und Basen, sowie eingesetzte Temperaturen und die Ausbeuten.

| Methylester der | Lösemittel | Base | Reakt.-Temp. | Ausbeute DBM |
|---|---|---|---|---|
| Benzoesäure | Ether | NaH/EtOH | 15 °C | 5% |
| Benzoesäure | Essovarsol®* | NaOMe | 120 °C | 64% |
| Benzoesäure | Isopar H®* | NaOMe | 120 °C | 75% |
| Benzoesäure | Solvesso 100®* | NaOMe | 120 °C | 86% |
| Benzoesäure | Toluol | NaOMe | 100 °C | 85% |
| Benzoesäure | Xylol | NaOMe | 125 °C | 95% |
| Benzoesäure | Tetrahydrofuran | K/BuOH | 5 °C | 60% |
| Stearinsäure | Xylol | NaOMe | 125 °C | 85% |
| Stearinsäure | Tetrahydrofuran | NaOMe | 5 °C | 57% |
| Stearinsäure | Dioxan | NaOMe | 30 °C | 69% |

| | | | | |
|---|---|---|---|---|
| * kommerziell erhältliche Benzinfraktionen der Firmen Esso bzw. Exxon | | | | |

### Beispiel

Unter N₂-Atmosphäre werden 1100 g wasserfreiem Xylol 270 g (1.50 mol) 30%-ige Natriummethanolatlösung (in Methanol) sowie 204 g (1.50 mol) Methylbenzoat gelöst und nach Abdestillieren des Methanols bei 125 °C portionsweise mit 150 g (1.25 mol) Acetophenon versetzt. Nach Abdestillieren des entstehenden Methanols, Versetzen der Mischung mit 390 g 20%-iger Schwefelsäure und Abtrennen der Wasserphase isoliert man 1447 g (95%) einer 18%-igen DBM-Lösung in Xylol.
- Analytische Daten der DBM-Lösung:: 18% DBM (Ausbeute: 95% DBM)
< 5% Benzoesäure
< 2% eingesetzte Edukte
in Xylol als Lösemittel

## Patentansprüche

1. Verfahren zur Herstellung von linearen 1,3-Diketonen der allgemeinen Formel (I) 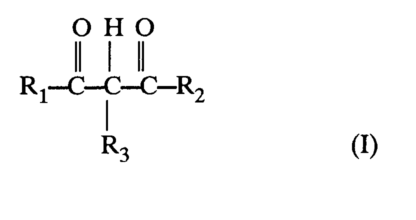 worin
R₁ und R₂ unabhängig voneinander für C₁-C₂₀-Alkyl, Phenyl, durch Halogen, Hydroxy, NO₂, C₁-C₄-Alkyl und/oder C₁-C₄-Alkoxy substituiertes Phenyl, C₇-C₉-Phenylalkyl oder für einen Rest der Formel (II) stehen
-A-X-R₄ (II)
wobei
A C₁-C₁₂-Alkylen, Phenylen, durch Halogen, Hydroxy, NO₂, C₁-C₄-Alkyl und/oder C₁-C₄-Alkoxy substituiertes Phenylen oder durch Hydroxy, Halogen oder/und Alkoxy substituiertes C₁-C₁₂-Alkylen bedeutet,
X für Sauerstoff oder Schwefel steht und
R₄ Wasserstoff, C₁-C₁₈-Alkyl, Phenyl, durch Halogen, Hydroxy, C₁-C₄-Alkyl, NO₂ und/oder C₁-C₄-Alkoxy substituiertes Phenyl oder C₇-C₉-Phenylalkyl bedeutet und
R₃ Wasserstoff, C₁-C₂₀-Alkyl, Phenyl, durch Halogen, Hydroxy, C₁-C₄-Alkyl, NO₂ und/oder C₁-C₄-Alkoxy substituiertes Phenyl oder C₇-C₉-Phenylalkyl darstellt, durch Claisen-Kondensation von Ketonen der Formel (III) 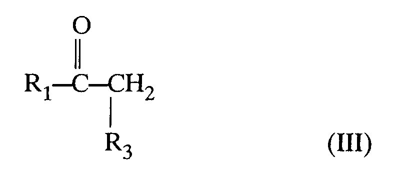 mit Estern der Formel (IV) 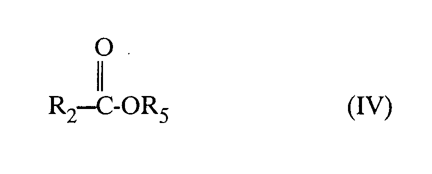 worin R₅ für C₁-C₅-Alkyl, Phenyl, oder durch Halogen, C₁-C₄-Alkyl oder Hydroxy substituiertes Phenyl steht; oder, wenn R₂ in Formel (I) -(CH₂)ₘOH bedeutet, auch mit cyclischen Estern der Formel (V) 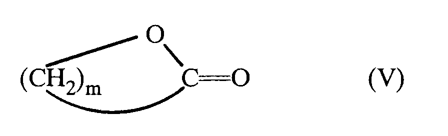 in der m 2 bis 10 bedeutet,
in Gegenwart eines Alkali- oder Erdalkalimetallhydrids oder C₁-C₅-Alkali- oder Erdalkalimetallalkoholats als Base und in einem inerten Lösungsmittel oder Lösungsmittelgemisch durchführt, **dadurch gekennzeichnet, daß** die Claisen-Kondensation bei einer Temperatur im Bereich von 95 - 150 °C durchgeführt wird.

2. Verfahren nach Anspruch 1, dadurch gekennzeichnet, daß das gebildete R₅-OH aus dem Reaktionsgemisch entfernt, vorzugsweise abdestilliert wird.

3. Verfahren nach Anspruch 1 oder 2, wobei das gebildete Salz in die freie Säure der Formel (I) überführt wird, vorzugsweise durch Behandlung des Reaktionsgemisches mit einer starken Säure, insbesondere mit Schwefelsäure,
und wobei vorzugsweise das durch die Bildung der freien Säure entstandene Nebenprodukt von dem organischen Reaktionsprodukt getrennt wird.

4. Verfahren nach Anspruch 2 und 3, dadurch gekennzeichnet, daß das entstandene organische Reaktionsprodukt ohne Hochreinigung bzw. Isolierung als ungereinigtes organisches Reaktionsprodukt gewonnen wird.

5. Verfahren nach Anspruch 4, dadurch gekennzeichnet, daß das ungereinigte organische Reaktionsprodukt (1,3-Diketon der Verbindung I), insbesondere als Losung in Xylol, mit weiteren Stabitisatorkomponenten zu einem Flüssigstabilisator verarbeitet wird und/oder daß diesem ungereinigten organischen Reaktionsprodukt ein chelatbildendes Metallsalz und gegebenenfalls ein Lösungsmittel, insbesondere Toluol oder Xylol, zugesetzt werden.

6. Verfahren nach Anspruch 5, dadurch gekennzeichnet, daß als chelatbildendes Metallsalz eines oder mehrere Metalle der Alkali-, Erdalkalireihe bzw. der 3. Hauptgruppe und 2. Nebengruppe des periodischen Systems eingesetzt werden.

7. Verfahren nach Anspruch 5 oder 6, dadurch gekennzeichnet, daß das chelatbildende Metallsalz in einer Menge zugesetzt wird derart, daß das Mol-Verhältnis von chelatbildendem Metallsalz zu 1,3-Diketon im Bereich 0:1 - 1:3,3 liegt.

8. Verfahren nach einem der vorhergehenden Ansprüche, dadurch gekennzeichnet, daß R₁ und R₂ unabhängig voneinander C₁-C₂₀-Alkyl, Phenyl, (C₁-C₄-Alkyl)-phenyl oder einen Rest der Formel (II) bedeuten,
A für C₁-C₆-Alkylen steht,
R₄ Wasserstoff, C₁-C₁₈-Alkyl, Phenyl oder (C₁-C₄-Alkyl)-phenyl darstellt und
R₃ für Wasserstoff und C₁-C₄-Alkyl steht,
insbesondere worin
R₁ und R₂ unabhängig voneinander C₁-C₁₈-Alkyl, Phenyl oder einen Rest der Formel (II) bedeuten,
R₄ Wasserstoff, Phenyl oder C₁-C₁₈-Alkyl darstellt und
R₃ für Wasserstoff steht,
vorzugsweise
worin R₁ und R₂ Phenyl oder einen Rest der Formel (II) bedeuten, R₄ Phenyl und X O bedeuten.

9. Verwendung des organischen Teils einer Claisen-Kondensation, wie sie im Oberbegriff des Anspruchs 1 definiert ist, ohne Hochreinigung als Co-Stabilisator für Kunststoffe.

10. Verwendung nach Anspruch 9, dadurch gekennzeichnet, daß das ungereinigte organische Reaktionsprodukt hergestellt nach einem der Ansprüche 1 bis 8 eingesetzt wird.

11. Verwendung nach Anspruch 9 oder 10, dadurch gekennzeichnet, daß der ungereinigte organische Teil der Claisen-Kondensation zusammen mit einem chelatbildenden Metallsalz, insbesondere einem solchen eines oder mehrerer der Metalle der Alkali-, Erdalkalireihe bzw. der 3. Hauptgruppe und 2. Nebengruppe des periodischen Systems, verwendet wird.

12. Verwendung nach einem der Ansprüche 9 bis 11, dadurch gekennzeichnet, daß der ungereinigte organische Teil der Claisen-Kondensation als Co-Stabilisator für halogenhaltige Kunststoffe, insbesondere chlorhaltige Kunststoffe, vorzugsweise PVC, eingesetzt wird.

13. Flüssigstabilisator geeignet für die Stabilisierung von Kunststoffen, insbesondere halogenhaltigen Kunststoffen, vorzugsweise PVC, dadurch gekennzeichnet, daß dieser einen durch Mischung des organischen Teils einer Claisen-Kondensation, wie sie im Oberbegriff des Anspruchs 1 definiert ist, ohne Hochreinigung entstanden ist, mit einem chelatbildenden Metallsalz enthält.

14. Flüssigstabilisator nach Anspruch 13, dadurch gekennzeichnet, daß dieser folgende Bestandteile aufweist:
1 - 5 Gewichtsteile ungereinigter organischer Teil der Claisen-Kondensation (1,3-Diketon),
5 - 50 Gewichtsteile einer Ca- oder Ba-Seife einer C₈-C₁₈-Carbonsäure,
2 - 35 Gewichtsteile einer Zn-Seife einer C₈-C₁₈-Carbonsäure,
0 - 55 Gewichtsteile eines organischen Phosphits,
ggf. Epoxide, Antioxidantien, Weichmacher, Losemittel.

15. Flüssigstabilisator nach einem der Ansprüche 13 oder 14, dadurch gekennzeichnet, daß er als chelatbildendes Metallsalz ein solches eines oder mehrerer der Metalle der Alkali-, Erdalkalireihe bzw. der 3. Hauptgruppe und 2. Nebengruppe des periodischen Systems enthält.

16. Flüssigstabilisator nach einem der Ansprüche 13 bis 15, dadurch gekennzeichnet, daß das Mol-Verhältnis von eingesetztem chelatbildenden Metallsalz zu 1,3-Diketon 0:1 - 1:3,3 beträgt.

17. Kunststoffmaterial enthaltend einen Kunststoff, insbesondere einen halogenhaltigen Kunststoff, vorzugsweise PVC, und zumindest einen Flüssigstabilisator, gekennzeichnet durch einen Gehalt eines Flüssigstabilisators gemäß einem der Ansprüche 13 bis 16.

18. Kunststoffmaterial nach Anspruch 17, dadurch gekennzeichnet, daß der Flüssigstabilisator gemäß einem der Ansprüche 13 bis 16 in einer Menge von 1 - 7 Gewichtsteile bezogen auf 100 Gewichtsteile Kunststoff vorliegt.

19. Kunststoffmaterial nach einem der Ansprüche 17 oder 18, dadurch gekennzeichnet, daß der Flüssigstabilisator Dibenzoylmethan und/oder Stearoyl-benzoylmethan enthält.
